# FASCICULE DE BREVET EUROPEEN

(11) **EP 2 114 378 B1**
(45) Date de publication et mention de la délivrance du brevet: **30.08.2017**
(21) Numéro de dépôt: 08702361.0
(22) Date de dépôt: 30.01.2008
(51) Int. Cl.: A61K 9/16

(54) **PROCEDE POUR METTRE EN OEUVRE DES MATIERES ACTIVES AFIN DE LES PROTEGER ET D'OPTIMISER LEUR MODE DE DELIVRANCE**
VERFAHREN ZUR REALISIERUNG VON WIRKSTOFFEN ZU DEREN SCHUTZ UND ZUR OPTIMIERUNG IHRES ABGABEMODUS
METHOD FOR REALISING ACTIVE INGREDIENTS IN ORDER TO PROTECT THEM AND OPTIMISE THEIR DELIVERY MODE

(30) Priorité: 09.02.2007 FR 0700926
(43) Date de publication de la demande: 11.11.2009
(73) Titulaire: COATEX, 69730 Genay (FR)
(72) Inventeur: GUERRET, Olivier, F-69890 La Tour De Salvagny (FR); SUAU, Jean-Marc, F-69480 Lucenay (FR)
(74) Mandataire: Balmefrezol, Ludovic Francis Pierre
(86) Numéro de dépôt international: PCT/IB2008/000261
(87) Numéro de publication internationale: WO 2008/096237

(56) Documents cités:
- EP-A- 1 331 262
- US-A- 5 441 660
- US-B1- 6 372 259

## Description

### Résumé de l'invention

L'invention concerne une nouvelle méthode pour formuler des principes actifs pharmaceutiques de manière à les protéger lors de l'ingestion orale et permettre de contrôler la libération du principe actif pharmaceutique dans l'intestin. Elle repose sur l'utilisation d'émulsions acryliques et épaississantes à pH supérieur à 5, contenant des groupements hydrophobes, pour encapsuler des principes actifs pharmaceutiques dans le but de favoriser leur franchissement de la barrière gastro-intestinale tout en maîtrisant la cinétique de libération desdits principes actifs.

A ce titre, un premier objet de l'invention est un procédé de fabrication d'une formulation contenant un principe actif pharmaceutique, par mélange d'eau, d'une émulsion de type HASE, d'un principe actif pharmaceutique, ledit mélange ayant un pH ajusté à une valeur supérieure à 5 selon les revendications On peut ensuite faire précipiter ce mélange à un pH inférieur à 3 : on obtient alors une dispersion dans l'eau de particules solides, constituées d'un principe actif pharmaceutique encapsulé dans des particules de polymères. On peut enfin éventuellement purifier cette dispersion, en vue d'obtenir des particules solides, constituées d'un principe actif pharmaceutique encapsulé dans des particules de polymères. Un dernier objet de l'invention consiste en l'utilisation de ces formulations aqueuses, de ces dispersions dans l'eau de particules solides et de ces particules solides, comme agents ayant la double fonction de protéger un principe actif pharmaceutique en milieu acide et de le relarguer en milieu basique ou alcalin.

La Demanderesse indique que la présente Demande n'entend protéger d'aucune manière une méthode de traitement thérapeutique. La présente Demande se rapporte uniquement à un procédé de fabrication de formulations contenant un principe actif pharmaceutique, aux formulations ainsi obtenues et à leur utilisation avec la double fonction particulière de protéger un principe actif pharmaceutique en milieu acide et de le relarguer en milieu basique ou alcalin : la fonction ici protégée n'est en aucun cas la fonction thérapeutique du principe actif.

### Définitions préalables

Principe actif pharmaceutique : dans le contexte de cette invention, nous désignons par ce terme toute substance ayant une action thérapeutique. Pour une meilleure compréhension, on pourra utiliser dans la Demande l'expression écourtée de "principe actif", en vue de désigner le "principe actif pharmaceutique".

HASE : acronyme anglosaxon pour Hydrophobically Alkali Swellable Emulsion. Ce terme désigne des épaississants acryliques à base d'acide (méth)acrylique, d'un ester de ces acides et d'un monomère hydrophobe.

### Problème technique et art antérieur

Si l'activité d'un principe actif pharmaceutique est liée à sa structure chimique, son efficacité dépend prioritairement de sa concentration disponible pour agir dans l'organisme. Le mode de diffusion dans l'organisme de ce principe actif est donc un facteur clé pour déterminer l'efficacité d'une formule comprenant ce principe actif. Le galéniste choisira donc un mode d'administration qui permettra de préserver au maximum l'intégrité du principe actif dans l'organisme.

Les modes d'administration sont variés mais dans le but d'améliorer le confort des malades, le mode préféré d'administration est la voie orale. Cependant, le principe actif doit alors passer la barrière gastro-intestinale. Si le principe actif peut être modifié par des réactions chimiques ayant lieu dans le système digestif, le galéniste devra surdoser le principe actif dans la formulation ce qui est non seulement économiquement négatif mais ce qui peut aussi entraîner des effets secondaires indésirables. Il est donc important de fournir au galéniste des moyens de protéger les principes actifs pour leur permettre de passer au mieux la barrière gastro-intestinale.

Une méthode connue de l'homme du métier réside dans la mise en oeuvre de copolymères blocs hydrophile-hydrophobe qui conduisent à des structures micellaires de type coeur-écorce pouvant enfermer un principe actif. Ces structures sont notamment décrites dans le document WO2004 / 112757 et réagissent au pH du milieu dans lequel elles sont placées. Ces solutions ne conviennent pas puisqu'elles reposent sur la mise en oeuvre de solvants pour incorporer un principe actif hydrophobe au sein des micelles (voir notamment l'exemple 1 du document pré-cité). Or, l'utilisation de solvants pose des problèmes d'un point de vue industriel (danger d'explosion dans les ateliers) et sanitaire (éventuelles traces de solvant résiduel). US 6372259 concerne des complexes polymériques comprenant du polyvinyl acetate phthalate (PVAP) et du polyvinylpyrrolidone (PVP), permettant d'encapsuler un médicament. Une autre méthode consiste en l'utilisation de polymères acryliques, éventuellement fabriqués en émulsion tels que des émulsions épaississantes acryliques plus connues sous la dénomination de ASE (selon l'expression anglaise Alcali Swellable Emulsion) pour vernir des dragées contenant un principe actif. Ce vernis se dissout dans l'eau à pH élevé (de 5 à 7 selon les structures), ce qui permet un délitage de la formulation dans les parties de l'intestin qui présentent ces conditions de pH. De tels polymères sont commercialement disponibles sous les appellations Eudragit™, ou Kollicoat™, Eastacryl 30D™. Cette méthode présente le désavantage de nécessiter une préparation préalable de la dragée avant de la vernir avec une solution neutralisée de ASE. Or, les principes actifs pharmaceutiques ayant souvent un caractère hydrophobe, il faut souvent les formuler dans un solvant avant de les incorporer dans une dragée, et on s'expose alors aux dangers précédemment évoqués.

Une des originalités du procédé selon l'invention est d'utiliser des HASE, différentes des ASE par le fait que leur structure contient un monomère hydrophobe associatif. Ce monomère possède la propriété, lorsque l'émulsion est épaissie à pH élevé, de créer des interactions hydrophobes associatives qui renforcent l'effet épaississant par rapport à un polymère n'ayant pas en son sein de tels monomères. Ces nodules hydrophobes dont la taille varie entre 5 et 100 nanomètres sont autant de cages de solvatation d'un principe actif hydrophobe. La dilution d'un principe actif dans une solution de HASE est donc facilitée et ne nécessite pas le recours à un solvant intermédiaire.

Les solutions ainsi obtenues à pH alcalin ou légèrement acide (> 5) sont susceptibles d'être déstabilisées par un changement de pH. Ainsi, le passage d'une telle solution par l'estomac dont le pH est proche de 1 provoque une séparation de phase des polymères entraînant le principe actif au sein des particules de polymère. Cette séparation de phase peut correspondre à une précipitation lorsque la température de transition vitreuse du polymère de l'émulsion est suffisamment élevée. Lorsque ces particules transitent dans l'intestin, elles passent du duodénum (pH de 6) vers l'ileum (pH de 7,4). Au fur et à mesure de cette remontée du pH, lesdites particules retrouvent progressivement une hydrosolubilité qui permet le relarguage du principe actif : celui-ci peut alors franchir la paroi intestinale.

Un des mérites de la Demanderesse est d'avoir su identifier et utiliser le phénomène de structuration de l'eau via une émulsion de type HASE, selon les revendications, à pH supérieur à 5 : on protège ainsi naturellement les principes actifs dissous dans cette solution par effondrement de la structure dans le milieu acide de l'estomac. Le principe actif se retrouve en effet au coeur de nodule nanométrique dans les particules de polymère précipitées, ces particules ayant des tailles micrométriques.

Une telle mise en oeuvre des émulsions de type HASE est dans l'état de nos connaissances actuelles une utilisation nouvelle de ces objets largement décrits dans des applications pour la peinture (voir les documents FR 2 693 203, FR 2 872 815, FR 2633 930), le domaine des cosmétiques (voir le document FR 2 872 815 déjà cité) ou encore le secteur des bétons (voir la demande de brevet française non encore publiée et portant le numéro de dépôt FR 07 00086). En outre, ces domaines techniques sont très éloignés de celui relatif à la présente invention, et les documents précités ne divulguent ni n'enseignent rien qui aurait pu orienter l'homme du métier vers la présente invention.

La description de la formulation peut varier en fonction de la viscosité du mélange : pour des solutions faiblement épaissies, la formulation pourra être décrite comme un sirop ; pour des formulation gélifiées, on pourra façonner ces solutions sous forme de gélules par exemple.

Un autre avantage du procédé selon la présente invention est de délivrer un principe actif sous une forme qui le protège ou le libère, en fonction du pH de son environnement, cette forme pouvant être triple :
- celle d'un liquide qui est une solution aqueuse, lorsque le produit est préparé en ne réalisant que l'étape de mélange du procédé selon l'invention à un pH supérieur à 5 (la précipitation a alors lieu dans l'estomac du patient),
- celle d'un liquide qui est une dispersion dans l'eau de particules solides, lorsque la préparation du produit met aussi en oeuvre l'étape de précipitation à un pH inférieur à 3,
- celle d'un solide constitué des particules solides du principe actif qui a été piégé dans les particules de polymères, lorsqu'on a mis en oeuvre l'étape de purification du procédé selon l'invention.

### Description de l'invention :

Un premier objet de l'invention est donc un procédé de fabrication d'une formulation contenant un principe actif pharmaceutique, et caractérisé en ce qu'il comprend les étapes de :
a) mélange d'une émulsion de type HASE, d'un principe actif pharmaceutique et d'eau, ledit mélange ayant un pH supérieur à 5, préférentiellement 6, très préférentiellement 7, la dite émulsion de type HASE contenant au moins un copolymère de : l'acide (méth)acrylique, un monomère non hydrosoluble ester (méth)acrylique, et un monomère contenant au moins un groupement hydrophobe possédant la formule générale selon les revendications.
b) précipitation par ajustement du pH à une valeur inférieure à 3, préférentiellement 2, en vue d'obtenir une dispersion dans l'eau de particules solides,
c) éventuellement purification, en vue d'obtenir des particules solides.

Le procédé selon l'invention est aussi caractérisé en ce que le pH du mélange, au cours de l'étape a), est ajusté au moyen d'une base organique ou minérale. Pratiquement, les constituants (principe actif, eau, émulsion de type HASE, ainsi que la base minérale ou organique) sont introduits sous agitation dans un réacteur; l'ordre d'introduction sera choisi par l'homme du métier, notamment en fonction de la solubilité dans l'eau du principe actif à encapsuler.

Le procédé selon l'invention est aussi caractérisé en ce qu'on met en oeuvre, dans l'étape a), de 0,1 % à 20 %, préférentiellement de 0,1 % à 10 %, très préférentiellement de 0,1 % à 5 % en poids sec d'une émulsion de type HASE, par rapport au poids total de la formulation aqueuse obtenue après l'étape a).

Le procédé selon l'invention est aussi caractérisé en ce qu'on met en oeuvre, dans l'étape a), de 0,1 % à 20 % en poids sec d'un principe actif pharmaceutique qui est hydrophile ou hydrophobe, par rapport au poids total de la formulation aqueuse obtenue après l'étape a).

Le procédé selon l'invention est aussi caractérisé en ce qu'on met en oeuvre un acide moyennement fort ou fort au cours de l'étape b).

Le procédé selon l'invention est aussi caractérisé en ce que l'émulsion de type HASE contient au moins un copolymère de l'acide (méth)acrylique, d'un monomère non hydrosoluble qui est préférentiellement un ester (méth)acrylique choisi très préférentiellement parmi l'acrylate d'éthyle, l'acrylate de butyle, le méthacrylate de méthyle et leurs mélanges, et d'un monomère contenant au moins un groupement hydrophobe.

Le procédé selon l'invention est aussi caractérisé en ce que ledit monomère contenant au moins un groupement hydrophobe possède la formule générale : 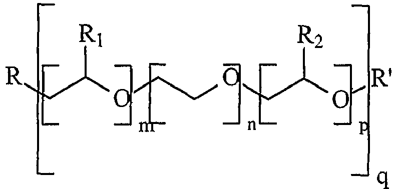 où :
- m, n, p et q sont des entiers et m, n, p sont inférieurs à 150,
- R comporte une fonction vinylique polymérisable,
- R₁ et R₂ sont identiques ou différents et représentent des atomes d'hydrogène ou des groupements alkyls,
- R' est un groupement hydrophobe comportant au moins 6, préférentiellement au moins 10, très préférentiellement au moins 12 atomes de carbone.

Le procédé selon l'invention est aussi caractérisé en ce que le principe actif est choisi parmi les antidouleurs et préférentiellement parmi l'aspirine, l'ibuprofène, la codéine ou la morphine, ou est choisi parmi les principes actifs de sevrage et préférentiellement parmi la buprénorphine ou la méthadone, ou choisi parmi les antibiotiques et préférentiellement parmi la pénicilline, l'érythromycine, la roxithromycine ou la télithromycine, ou choisi parmi les anticancéreux et préférentiellement parmi la capécitabine ou l'étoposide, ou choisi parmi les principes actifs utilisés dans le traitement du diabète et est préférentiellement l'insuline, ou choisi parmi les principes actifs utilisés comme antidépresseurs et est préférentiellement la benzodiazépine, ou choisi parmi les principes actifs utilisés dans le traitement des maladies gastriques et est préférentiellement l'esomeprazole, ou choisi parmi les principes actifs utilisés pour le contrôle du choléstérol et est préférentiellement le simvastatin, ou choisi parmi les principes actifs utilisés dans le traitement des maladies cardiovasculaires et préférentiellement parmi le lipitor ou l'amlodipine, ou choisi parmi les principes actifs utilisés dans le traitement des maladies mentales et préférentiellement parmi l'olanzapine ou la risperidone, ou parmi les mélanges de ces principes actifs.

Un autre objet de l'invention est constitué de la formulation aqueuse contenant un principe actif pharmaceutique, et obtenue par la mise en oeuvre de l'étape a) du procédé précédemment décrit.

Cette formulation aqueuse contenant un principe actif pharmaceutique est caractérisée :
a) en ce qu'elle contient de l'eau, une émulsion de type HASE et un principe actif pharmaceutique,
b) et en ce qu'elle possède un pH supérieur à 5, préférentiellement 6, très préférentiellement 7.

Cette formulation aqueuse est aussi caractérisée en ce qu'elle contient de 0,1 % à 20 %, préférentiellement de 0,1 % à 10 %, très préférentiellement de 0,1 % à 5 % en poids sec d'une émulsion de type HASE, par rapport à son poids total.

Cette formulation aqueuse est aussi caractérisée en ce qu'elle contient de 0,1 % à 20 % en poids sec d'un principe actif pharmaceutique qui est hydrophile ou hydrophobe, par rapport à son poids total.

Cette formulation aqueuse est aussi caractérisée en ce que l'émulsion de type HASE contient au moins un copolymère de l'acide (méth)acrylique, d'un monomère non hydrosoluble qui est préférentiellement un ester (méth)acrylique choisi très préférentiellement parmi l'acrylate d'éthyle, l'acrylate de butyle, le méthacrylate de méthyle et leurs mélanges, et d'un monomère contenant au moins un groupement hydrophobe possédant la formule générale : 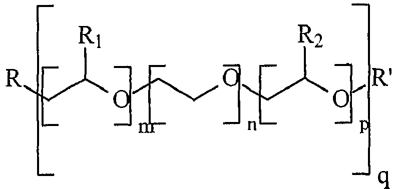 où :
- m, n, p et q sont des entiers et m, n, p sont inférieurs à 150,
- R comporte une fonction vinylique polymérisable,
- R₁ et R₂ sont identiques ou différents et représentent des atomes d'hydrogène ou des groupements alkyls,
- R' est un groupement hydrophobe comportant au moins 6, préférentiellement au moins 10, très préférentiellement au moins 12 atomes de carbone.

Cette formulation aqueuse est aussi caractérisée en ce que le principe actif est choisi parmi les antidouleurs et préférentiellement parmi l'aspirine, l'ibuprofène, la codéine ou la morphine, ou est choisi parmi les principes actifs de sevrage et préférentiellement parmi la buprénorphine ou la méthadone, ou choisi parmi les antibiotiques et préférentiellement parmi la pénicilline, l'érythromycine, la roxithromycine ou la télithromycine, ou choisi parmi les anticancéreux et préférentiellement parmi la capécitabine ou l'étoposide, ou choisi parmi les principes actifs utilisés dans le traitement du diabète et est préférentiellement l'insuline, ou choisi parmi les principes actifs utilisés comme antidépresseurs et est préférentiellement la benzodiazépine, ou choisi parmi les principes actifs utilisés dans le traitement des maladies gastriques et est préférentiellement l'esomeprazole, ou choisi parmi les principes actifs utilisés pour le contrôle du choléstérol et est préférentiellement le simvastatin, ou choisi parmi les principes actifs utilisés dans le traitement des maladies cardiovasculaires et préférentiellement parmi le lipitor ou l'amlodipine, ou choisi parmi les principes actifs utilisés dans le traitement des maladies mentales et préférentiellement parmi l'olanzapine ou la risperidone, ou parmi les mélanges de ces principes actifs.

Un autre objet de l'invention consiste en la formulation constituée de particules solides en dispersion dans l'eau, et obtenue par mise en oeuvre de l'étape de précipitation b) du procédé auparavant décrit.

Cette dispersion de particules solides dans l'eau est caractérisée en ce que les particules solides qui la constituent, contiennent un principe actif pharmaceutique et un copolymère de l'acide (méth)acrylique, d'un monomère non hydrosoluble qui est préférentiellement un ester (méth)acrylique choisi très préférentiellement parmi l'acrylate d'éthyle, l'acrylate de butyle, le méthacrylate de méthyle et leurs mélanges, et d'un monomère contenant au moins un groupement hydrophobe possédant la formule générale : 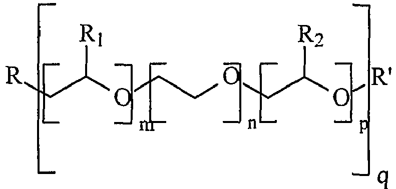 où :
- m, n, p et q sont des entiers et m, n, p sont inférieurs à 150,
- R comporte une fonction vinylique polymérisable,
- R₁ et R₂ sont identiques ou différents et représentent des atomes d'hydrogène ou des groupements alkyls,
- R' est un groupement hydrophobe comportant au moins 6, préférentiellement au moins 10, très préférentiellement au moins 12 atomes de carbone.

Cette dispersion de particules solides dans l'eau est aussi caractérisée en ce que le principe actif est choisi parmi les antidouleurs et préférentiellement parmi l'aspirine, l'ibuprofène, la codéine ou la morphine, ou est choisi parmi les principes actifs de sevrage et préférentiellement parmi la buprénorphine ou la méthadone, ou choisi parmi les antibiotiques et préférentiellement parmi la pénicilline, l'érythromycine, la roxithromycine ou la télithromycine, ou choisi parmi les anticancéreux et préférentiellement parmi la capécitabine ou l'étoposide, ou choisi parmi les principes actifs utilisés dans le traitement du diabète et est préférentiellement l'insuline, ou choisi parmi les principes actifs utilisés comme antidépresseurs et est préférentiellement la benzodiazépine, ou choisi parmi les principes actifs utilisés dans le traitement des maladies gastriques et est préférentiellement l'esomeprazole, ou choisi parmi les principes actifs utilisés pour le contrôle du choléstérol et est préférentiellement le simvastatin, ou choisi parmi les principes actifs utilisés dans le traitement des maladies cardiovasculaires et préférentiellement parmi le lipitor ou l'amlodipine, ou choisi parmi les principes actifs utilisés dans le traitement des maladies mentales et préférentiellement parmi l'olanzapine ou la risperidone, ou parmi les mélanges de ces principes actifs.

Un autre objet de l'invention réside dans la formulation constituée des particules solides obtenues par mise en oeuvre de l'étape de purification c) du procédé auparavant décrit.

Ces particules solides sont caractérisées en ce qu'elles contiennent un principe actif pharmaceutique et un copolymère de l'acide (méth)acrylique, d'un monomère non hydrosoluble qui est préférentiellement un ester (méth)acrylique choisi très préférentiellement parmi l'acrylate d'éthyle, l'acrylate de butyle, le méthacrylate de méthyle et leurs mélanges, et d'un monomère contenant au moins un groupement hydrophobe possédant la formule générale: 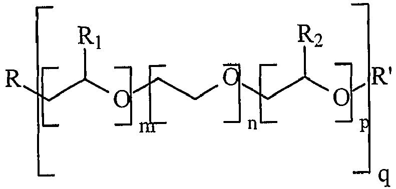 où :
- m, n, p et q sont des entiers et m, n, p sont inférieurs à 150,
- R comporte une fonction vinylique polymérisable,
- R₁ et R₂ sont identiques ou différents et représentent des atomes d'hydrogène ou des groupements alkyls,
- R' est un groupement hydrophobe comportant au moins 6, préférentiellement au moins 10, très préférentiellement au moins 12 atomes de carbone.

Cette particules solides sont aussi caractérisées en ce que le principe actif est choisi parmi les antidouleurs et préférentiellement parmi l'aspirine, l'ibuprofène, la codéine ou la morphine, ou est choisi parmi les principes actifs de sevrage et préférentiellement parmi la buprénorphine ou la méthadone, ou choisi parmi les antibiotiques et préférentiellement parmi la pénicilline, l'érythromycine, la roxithromycinz ou la télithromycinz, ou choisi parmi les anticancéreux et préférentiellement parmi la capécitabine ou l'étoposide, ou choisi parmi les principes actifs utilisés dans le traitement du diabète et est préférentiellement l'insuline, ou choisi parmi les principes actifs utilisés comme antidépresseurs et est préférentiellement la benzodiazépine, ou choisi parmi les principes actifs utilisés dans le traitement des maladies gastriques et est préférentiellement l'esomeprazole, ou choisi parmi les principes actifs utilisés pour le contrôle du choléstérol et est préférentiellement le simvastatin, ou choisi parmi les principes actifs utilisés dans le traitement des maladies cardiovasculaires et préférentiellement parmi le lipitor ou l'amlodipine, ou choisi parmi les principes actifs utilisés dans le traitement des maladies mentales et préférentiellement parmi l'olanzapine ou la risperidone, ou parmi les mélanges de ces principes actifs.

Un dernier objet de l'invention est l'utilisation des formulations aqueuses d'un principe actif pharmaceutique, des dispersions aqueuses de particules solides d'un principe actif pharmaceutique et des particules solides d'un principe actif pharmaceutique, comme agent ayant la double fonction de protéger un principe actif pharmaceutique en milieu acide et de le relarguer en milieu basique ou alcalin.

### EXEMPLES

### Exemple 1

Cet exemple illustre le procédé selon l'invention, dans lequel on réalise tout d'abord selon l'étape a) une formulation aqueuse d'un principe actif pharmaceutique qui est le bleu de méthylène et ce, en présence de différentes émulsions de type HASE, et à un pH supérieur à 5. Le bleu de méthylène est soluble dans l'eau et les solvants organiques, et sa couleur permettra de reconnaître s'il est encapsulé dans des particules de polymères, ou s'il est libéré dans l'eau.

On fait ensuite précipiter ces formulations selon l'étape b) du procédé de l'invention, par abaissement du pH à une valeur inférieure à 3 : on montre alors comment le bleu de méthylène se retrouve piégé dans des particules de polymères.

On les place enfin dans un milieu où on fait augmenter le pH, de manière à démontrer que le bleu de méthylène est alors relargué au fur et à mesure que le pH augmente. Cet exemple illustre donc les différents objets de l'invention et leur utilisation comme agents permettant de protéger un principe actif dans un environnement acide, et de le relarguer en milieu basique ou alcalin.

### Fabrication des formulations aqueuses

On pèse 3,33 g d'une émulsion de type HASE à 30 % d'extrait sec. On ajoute 100 g d'une solution d'eau contenant 130 ppm de bleu de méthylène. Le milieu est agité pendant cette addition, et le pH est ajusté à une valeur supérieure à 5 à partir d'une solution de soude. Un témoin ne contenant pas de HASE est également fabriqué par mélange de 0,013 g de bleu de méthylène dans 100 ml d'eau : son pH est ajusté lui aussi à une valeur supérieure à 5 par addition d'une solution de soude.

Les essais selon l'invention se caractérisent par le choix de l'émulsion, les viscosités Brookfield™ de la formulation à 10 et 100 tours par minute (notées respectivement µ₁₀ et µ₁₀₀) à 25°C : le rapport de ces viscosités est caractéristique des émulsions de type HASE et dépend des groupements hydrophobes choisis ainsi que des ratios monomériques, le niveau de viscosité atteint étant fonction de la quantité d'émulsion de type HASE utilisée. Ces résultats sont reportés dans le tableau 1.

**Tableau 1**

| **Essai n°** | **Emulsion HASE** | **pH** | **µ₁₀ (mPa.s)** | **µ₁₀₀ (mPa.s)** |
|---|---|---|---|---|
| témoin | - | 9,0 | 0 | 0 |
| 1 | Rhéo™ 2000 | 9,6 | 40 | 20 |
| 2 | Rhéo™ 2100 | 9,5 | 40 | 20 |
| 3 | Rhéo™ 3000 | 9,1 | 1000 | 340 |
| 4 | Rhéo™ 3800 | 7,3 | 1060 | 2300 |
| 5 | Thixol™ 53L | 7,4 | 5200 | 32000 |
| 6 | Viscoatex™ 730 | 11,2 | 790 | 3900 |
| 7 | Acrysol™ TT935 | 7,45 | 60 | 100 |
| 8 | Acrysol™ TT615 | 11,9 | 1210 | 7800 |

Les polymères des essais 1 à 6 sont des épaississants acryliques HASE commercialisés par la société COATEX, les polymères des essais 7 et 8 sont des émulsions de type HASE commercialisées par la société ROHM & HAAS.

Toutes les formulations aqueuses selon l'invention sont transparentes, uniformément bleues et les viscosités trouvées confirment bien la présence de gels (par rapport à la formulation témoin). Les dimensions de ces cages de solvatation du bleu de méthylène sont de l'ordre de quelques dizaines de nanomètres puisque la lumière n'est pas diffractée par cette solution. Leur répartition est homogène comme l'indique l'uniformité de la coloration bleue.

### Co-précipitation des formulations aqueuses

La concentration dans l'eau de bleu de méthylène est déterminée par spectrométrie UV en mesurant l'intensité d'absorption à une longueur d'onde de 652 nm. Le témoin sert à étalonner le dispositif : le pourcentage de lumière qu'il absorbe est fixé égal à 100 %. On prélève 40 g de chacune des solutions correspondant aux essais n° 1 à 8 et 40 g de la solution témoin. On coule goutte à goutte ces 40 g sur une solution à 15 % d'acide phosphorique de manière à ce que le pH final de la phase aqueuse soit égal à 1,2. Pour les essais n° 1 à 8, on observe une séparation de phase : il se forme des particules bleues et les surnageants sont très peu colorés. En revanche, le témoin conserve une couleur uniformément bleue.

La taille des particules obtenues a été mesurée par diffusion dynamique de la lumière à l'aide d'un Zétasizer™ nano S90 commercialisé par la société MALVERN™ (voir tableau 2).

**Tableau 2**

| Essai n° | Emulsion HASE | Taille des particules (nm) |
|---|---|---|
| 1 | Rhéo™ 2000 | 340 |
| 2 | Rhéo™ 2100 | 800 |
| 3 | Rhéo™ 3000 | 1260 |
| 4 | Rhéo™ 3800 | 4510 |
| 5 | Thixol™ 53L | 930 |
| 6 | Viscoatex™ 730 | 3200 |

### Libération du bleu de méthylène stimulée par le pH

La suspension obtenue dans le cadre de l'essai n° 2 est utilisée ensuite pour démontrer que dans un contexte de remontée du pH, les particules de polymères qui ont piégé le bleu de méthylène libèrent le principe actif. On ajoute donc goutte à goutte une solution 5 M de soude en maintenant la suspension sous agitation. Le dosage est réalisé en suivant une cinétique d'addition correspondant au gain d'une unité de pH par heure. Cette neutralisation modélise le cheminement d'un produit passant de l'estomac (pH=1) à l'ileum (pH=7,4).

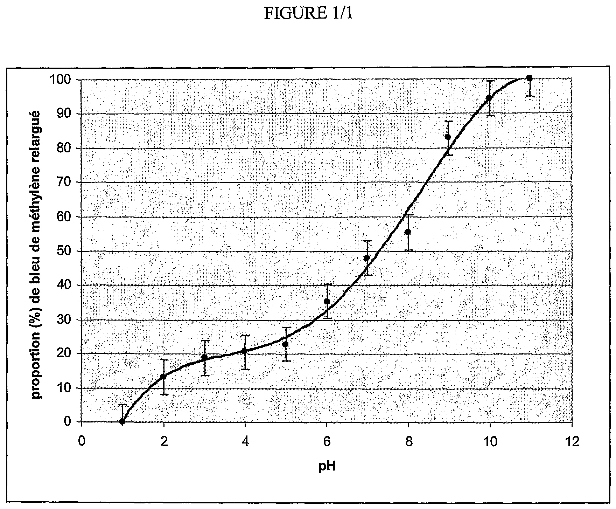

Au fur et à mesure de la neutralisation, on analyse le surnageant par réfractométrie : on détermine ainsi la quantité relative de bleu de méthylène par rapport à la quantité théorique. La figure 1/1 représente la proportion de bleu de méthylène libéré en fonction du pH. On démontre ainsi la capacité des vésicules formées à libérer leur principe actif en fonction du pH du milieu où elles sont en dispersion.

### Exemple 2

Cet exemple illustre le procédé selon l'invention dans lequel le principe actif pharmaceutique est le 2,9 dichloro-5,12 dihydroquino [2,3-b] acridine-7,14 dione ou 2,6 dichloquinacridone commercialisé par la société CIBA GEIGY™ sous le nom de Magenta Cinquasia RT-235-D. Il s'agit d'un colorant non hydrosoluble. Sa couleur rouge permet de savoir aisément si la molécule est présente ou non dans l'eau.

### Fabrication des formulations aqueuses

On pèse 3,33 g d'une émulsion de type HASE commercialisée par la société COATEX sous le nom de Thixol™ 53L et qui présente un extrait sec égal à 30 % de son poids. On ajoute 100 g d'une solution d'eau et 0,015 g de colorant et ce, sous agitation. Le colorant demeure sous forme dispersée.

Le milieu est alors neutralisé avec une solution de soude à 20 % jusqu'à pH 9 et on obtient un gel où la poudre de colorant dispersée est finalement totalement incorporée dans la formulation jusqu'à l'obtention d'une solution rouge homogène, exempte de toute particule insoluble.

On obtient des viscosités Brookfield™ à 10 et 100 tours par minute et à 25°C, respectivement égales à 13 000 mPa.s et 1 900 mPa.s.

### Co précipitation

On ajoute alors une solution d'acide phosphorique de concentration 15 %, de manière à obtenir un pH égal à 2,4.

On observe une séparation de phase. Il se forme des particules rouges. Les surnageants deviennent très peu colorés.

## Revendications

1. Procédé de fabrication d'une formulation contenant une émulsion de type HASE (Hydrophobically Alkali Swellable Emulsion) et un principe actif pharmaceutique, comprenant les étapes de :
a) mélange d'une émulsion de type HASE, d'un principe actif pharmaceutique et d'eau, ledit mélange ayant un pH supérieur à 5, ladite émulsion de type HASE contenant au moins un copolymère de :
- l'acide (méth)acrylique,
- un monomère non hydrosoluble ester (méth)acrylique, et
- un monomère contenant au moins un groupement hydrophobe possédant la formule générale : 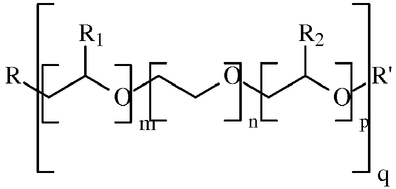 dans laquelle :
- m, n, p et q sont des entiers inférieurs à 150,
- R comporte une fonction vinylique polymérisable,
- R₁ et R₂ sont identiques ou différents et représentent H ou des groupements alkyls,
- R' est un groupement hydrophobe comportant au moins 6 atomes de carbone,
b) précipitation par ajustement du pH à une valeur inférieure à 3, en vue d'obtenir une dispersion dans l'eau de particules solides,
c) éventuellement purification, en vue d'obtenir des particules solides.

2. Procédé selon la revendication 1, selon lequel le pH du mélange, au cours de l'étape a), est ajusté au moyen d'une base organique ou minérale.

3. Procédé selon l'une des revendications 1 ou 2, selon lequel on met en oeuvre, dans l'étape a), de 0,1 % à 20 %, en poids sec de ladite émulsion de type HASE, par rapport au poids total de la formulation aqueuse obtenue après l'étape a).

4. Procédé selon l'une des revendications 1 à 3, selon lequel on met en oeuvre, dans l'étape a), de 0,1 % à 20 % en poids sec d'un principe actif pharmaceutique, hydrophile ou hydrophobe, par rapport au poids total de la formulation aqueuse obtenue après l'étape a).

5. Procédé selon l'une des revendications 1 à 4, **caractérisé en ce qu'**on met en oeuvre un acide moyennement fort ou fort au cours de l'étape b).

6. Procédé selon l'une des revendications 1 à 5, selon lequel le principe actif est choisi parmi les antidouleurs, les principes actifs de sevrage, les antibiotiques, les anticancéreux, les principes actifs utilisés dans le traitement du diabète, les antidépresseurs, les principes actifs utilisés dans le traitement des maladies gastriques, les principes actifs utilisés pour le contrôle du cholestérol, les principes actifs utilisés dans le traitement des maladies cardiovasculaires, les principes actifs utilisés dans le traitement des maladies mentales, ou un mélange de ces principes actifs.

7. Formulation aqueuse contenant de l'eau, un principe actif pharmaceutique et une émulsion de type HASE, ladite émulsion de type HASE contenant au moins un copolymère de :
- l'acide (méth)acrylique,
- un monomère non hydrosoluble ester (méth)acrylique, et
- un monomère contenant au moins un groupement hydrophobe possédant la formule générale : 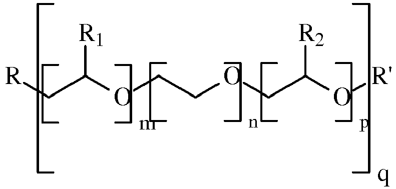 dans laquelle :
- m, n, p et q sont des entiers inférieurs à 150,
- R comporte une fonction vinylique polymérisable,
- R₁ et R₂ sont identiques ou différents et représentent H ou des groupements alkyls,
- R' est un groupement hydrophobe comportant au moins 6 atomes de carbone, ladite formulation possédant un pH supérieur à 5.

8. Formulation aqueuse selon la revendication 7, contenant de 0,1 % à 20 % en poids sec de ladite émulsion de type HASE, par rapport à son poids total.

9. Formulation aqueuse selon l'une des revendications 7 ou 8, contenant de 0,1 % à 20 % en poids sec d'un principe actif pharmaceutique, hydrophile ou hydrophobe, par rapport à son poids total.

10. Formulation aqueuse selon l'une des revendications 7 à 9, dans laquelle le principe actif est choisi parmi les antidouleurs, les principes actifs de sevrage, les antibiotiques, les anticancéreux, les principes actifs utilisés dans le traitement du diabète, les antidépresseurs, les principes actifs utilisés dans le traitement des maladies gastriques, les principes actifs utilisés pour le contrôle du cholestérol, les principes actifs utilisés dans le traitement des maladies cardiovasculaires, les principes actifs utilisés dans le traitement des maladies mentales, ou un mélange de ces principes actifs.

11. Dispersion de particules solides dans l'eau, lesdites particules solides contenant un principe actif pharmaceutique et un copolymère de :
- l'acide (méth)acrylique,
- un monomère non hydrosoluble ester (méth)acrylique, et
- un monomère contenant au moins un groupement hydrophobe possédant la formule générale : 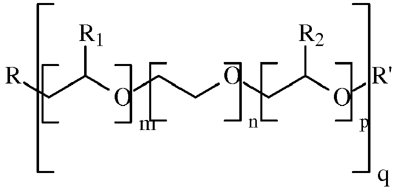 dans laquelle :
- m, n, p et q sont des entiers inférieurs à 150,
- R comporte une fonction vinylique polymérisable,
- R₁ et R₂ sont identiques ou différents et représentent H ou des groupements alkyls,
- R' est un groupement hydrophobe comportant au moins 6 atomes de carbone.

12. Dispersion de particules solides selon la revendication 11, dans laquelle le principe actif est choisi parmi les antidouleurs, les principes actifs de sevrage, les antibiotiques, les anticancéreux, les principes actifs utilisés dans le traitement du diabète, les antidépresseurs, les principes actifs utilisés dans le traitement des maladies gastriques, les principes actifs utilisés pour le contrôle du cholestérol, les principes actifs utilisés dans le traitement des maladies cardiovasculaires, les principes actifs utilisés dans le traitement des maladies mentales, ou un mélange de ces principes actifs.

13. Particules solides contenant un principe actif pharmaceutique et un copolymère de :
- l'acide (méth)acrylique,
- un monomère non hydrosoluble ester (méth)acrylique, et
- un monomère contenant au moins un groupement hydrophobe possédant la formule générale : 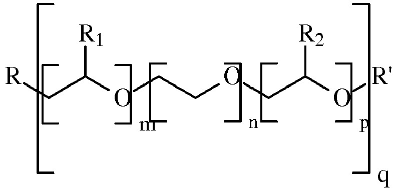 dans laquelle :
- m, n, p et q sont des entiers inférieurs à 150,
- R comporte une fonction vinylique polymérisable,
- R₁ et R₂ sont identiques ou différents et représentent H ou des groupements alkyls,
- R' est un groupement hydrophobe comportant au moins 6 atomes de carbone.

14. Particules solides selon la revendication 13, dans lesquelles le principe actif est choisi parmi les antidouleurs, les principes actifs de sevrage, les antibiotiques, les anticancéreux, les principes actifs utilisés dans le traitement du diabète, les antidépresseurs, les principes actifs utilisés dans le traitement des maladies gastriques, les principes actifs utilisés pour le contrôle du cholestérol, les principes actifs utilisés dans le traitement des maladies cardiovasculaires, les principes actifs utilisés dans le traitement des maladies mentales, ou un mélange de ces principes actifs.

15. Formulations aqueuses, dispersions aqueuses de particules solides ou particules solides, contenant un principe actif pharmaceutique et une émulsion de type HASE, ladite émulsion contenant au moins un copolymère de :
- l'acide (méth)acrylique,
- un monomère non hydrosoluble ester (méth)acrylique, et
- un monomère contenant au moins un groupement hydrophobe possédant la formule générale : 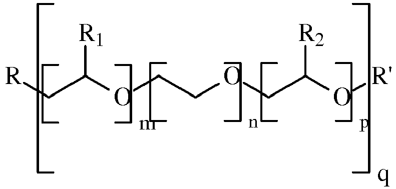 dans laquelle :
- m, n, p et q sont des entiers inférieurs à 150,
- R comporte une fonction vinylique polymérisable,
- R₁ et R₂ sont identiques ou différents et représentent H ou des groupements alkyls,
- R' est un groupement hydrophobe comportant au moins 6 atomes de carbone,
pour leur utilisation comme agent ayant la double fonction de protéger ledit principe actif pharmaceutique en milieu acide et de le relarguer en milieu basique ou alcalin.

16. Utilisation d'une émulsion de type HASE, ladite émulsion contenant au moins un copolymère de :
- l'acide (méth)acrylique,
- un monomère non hydrosoluble ester (méth)acrylique, et
- un monomère contenant au moins un groupement hydrophobe possédant la formule générale : 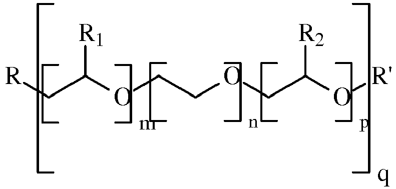 dans laquelle :
- m, n, p et q sont des entiers inférieurs à 150,
- R comporte une fonction vinylique polymérisable,
- R₁ et R₂ sont identiques ou différents et représentent H ou des groupements alkyls,
- R' est un groupement hydrophobe comportant au moins 6 atomes de carbone,
pour préparer une formulation aqueuse, une dispersion aqueuse de particules solides ou des particules solides, contenant un principe actif pharmaceutique.

## Patentansprüche

1. Verfahren zur Herstellung einer Formulierung, die eine Emulsion des HASE (Hydrophobically Alkali Swellable Emulsion) -Typs und einen pharmazeutischen Wirkstoff enthält, das die folgenden Schritte umfasst:
a) Mischen von einer Emulsion des HASE-Typs, einem pharmazeutischen Wirkstoff und Wasser, wobei das Gemisch einen pH-Wert über 5 hat, wobei die Emulsion des HASE-Typs mindestens ein Copolymer aus Folgendem enthält:
- (Meth)Acrylsäure,
- einem nicht-wasserlöslichen (Meth)Acrylsäureester-Monomer, und
- einem Monomer, das mindestens eine hydrophobe Gruppe mit der folgenden allgemeinen Formel enthält: 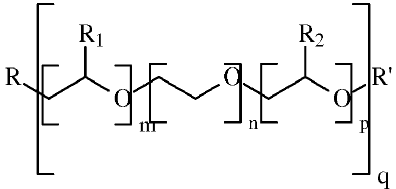 worin:
- m, n, p und q ganze Zahlen unter 150 sind,
- R eine polymerisierbare Vinylfunktion trägt,
- R₁ und R₂ gleich oder verschieden sind und für H oder Alkylgruppen stehen,
- R' eine hydrophobe Gruppe mit mindestens 6 Kohlenstoffatomen ist,
b) Ausfällung durch Einstellen des pH auf einen Wert unterhalb von 3, um eine Dispersion von festen Partikeln in Wasser zu erhalten,
c) gegebenenfalls Reinigung, um feste Partikel zu erhalten.

2. Verfahren nach Anspruch 1, wobei der pH-Wert des Gemischs während Schritt a), mithilfe einer organischen oder anorganischen Base eingestellt wird.

3. Verfahren nach einem der Ansprüche 1 oder 2, wobei man in Schritt a), von 0,1 % bis 20 % Trockengewicht der Emulsion des HASE-Typs, bezogen auf das Gesamtgewicht der nach Schritt a) erhaltenen wässrigen Zubereitung, einsetzt.

4. Verfahren nach einem der Ansprüche 1 bis 3, wobei man in Schritt a), von 0,1 % bis 20 % Trockengewicht eines hydrophilen oder hydrophoben pharmazeutischen Wirkstoffs, bezogen auf das Gesamtgewicht der nach Schritt a) erhaltenen wässrigen Formulierung, einsetzt.

5. Verfahren nach einem der Ansprüche 1 bis 4, **dadurch gekennzeichnet, dass** man eine mäßig starke oder starke Säure bei Schritt b) einsetzt.

6. Verfahren nach einem der Ansprüche 1 bis 5, wobei der Wirkstoff aus Schmerzmitteln, Entwöhnungswirkstoffen, Antibiotika, Antikrebsmitteln, zur Behandlung von Diabetes verwendeten Wirkstoffen, Antidepressiva, zur Behandlung von Magenerkrankungen verwendeten Wirkstoffen, zur Cholesterinkontrolle verwendeten Wirkstoffen, zur Behandlung von kardiovaskulären Erkrankungen verwendeten Wirkstoffen, zur Behandlung von psychischen Erkrankungen verwendeten Wirkstoffen oder einem Gemisch dieser Wirkstoffe ausgewählt wird.

7. Wässrige Formulierung, die Wasser, einen pharmazeutischen Wirkstoff und eine Emulsion des HASE-Typs umfasst, wobei die Emulsion des HASE-Typs mindestens ein Copolymer aus den Folgenden enthält:
- (Meth)Acrylsäure,
- einem nicht-wasserlöslichen (Meth)Acrylsäureester-Monomer, und
- einem Monomer, das mindestens eine hydrophobe Gruppe mit der folgenden allgemeinen Formel enthält: 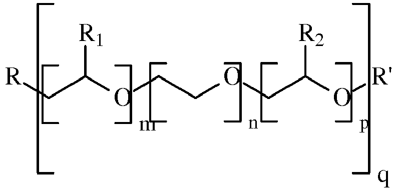 worin:
- m, n, p und q ganze Zahlen unter 150 sind,
- R eine polymerisierbare Vinylfunktion trägt,
- R₁ und R₂ gleich oder verschieden sind und für H oder Alkylgruppen stehen,
- R' eine hydrophobe Gruppe mit mindestens 6 Kohlenstoffatomen ist,
wobei die Formulierung einen pH-Wert über 5 besitzt.

8. Wässrige Formulierung nach Anspruch 7, die von 0,1 % bis 20 % Trockengewicht der Emulsion des HASE-Typs, bezogen auf ihr Gesamtgewicht, enthält.

9. Wässrige Formulierung nach einem der Ansprüche 7 oder 8, die von 0,1 % bis 20 % Trockengewicht eines hydrophilen oder hydrophoben pharmazeutischen Wirkstoffs, bezogen auf ihr Gesamtgewicht, umfasst.

10. Wässrige Formulierung nach einem der Ansprüche 7 bis 9, wobei der Wirkstoff aus Schmerzmitteln, Entwöhnungswirkstoffen, Antibiotika, Antikrebsmitteln, zur Behandlung von Diabetes verwendeten Wirkstoffen, Antidepressiva, zur Behandlung von Magenerkrankungen verwendeten Wirkstoffen, zur Cholesterinkontrolle verwendeten Wirkstoffen, zur Behandlung von kardiovaskulären Erkrankungen verwendeten Wirkstoffen, zur Behandlung von psychischen Erkrankungen verwendeten Wirkstoffen oder einem Gemisch dieser Wirkstoffe ausgewählt wird.

11. Dispersion fester Partikel in Wasser, wobei die festen Partikel einen pharmazeutischen Wirkstoff und ein Copolymer aus:
- (Meth)Acrylsäure,
- einem nicht-wasserlöslichen (Meth)Acrylsäureester-Monomer und
- einem Monomer, das mindestens eine hydrophobe Gruppe mit der folgenden allgemeinen Formel enthält: 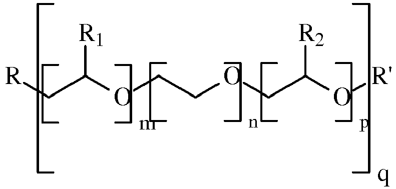 worin:
- m, n, p und q ganze Zahlen unter 150 sind,
- R eine polymerisierbare Vinylfunktion trägt,
- R₁ und R₂ gleich oder verschieden sind und für H oder Alkylgruppen stehen,
- R' eine hydrophobe Gruppe mit mindestens 6 Kohlenstoffatomen ist, enthalten.

12. Dispersion fester Partikel nach Anspruch 11, wobei der Wirkstoff aus Schmerzmitteln, Entwöhnungswirkstoffen, Antibiotika, Antikrebsmitteln, zur Behandlung von Diabetes verwendeten Wirkstoffen, Antidepressiva, zur Behandlung von Magenerkrankungen verwendeten Wirkstoffen, zur Cholesterinkontrolle verwendeten Wirkstoffen, zur Behandlung von kardiovaskulären Erkrankungen verwendeten Wirkstoffen, zur Behandlung von psychischen Erkrankungen verwendeten Wirkstoffen oder einem Gemisch dieser Wirkstoffe ausgewählt wird.

13. Feste Partikel, die einen pharmazeutischen Wirkstoff und ein Copolymer aus:
- (Meth)Acrylsäure,
- einem nicht-wasserlöslichen (Meth)Acrylsäureester-Monomer und
- einem Monomer, das mindestens eine hydrophobe Gruppe mit der folgenden allgemeinen Formel enthält: 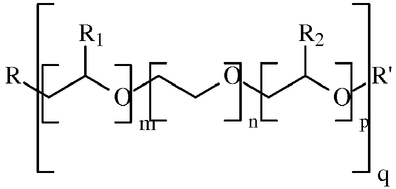 worin:
- m, n, p und q ganze Zahlen unter 150 sind,
- R eine polymerisierbare Vinylfunktion trägt,
- R₁ und R₂ gleich oder verschieden sind und für H oder Alkylgruppen stehen,
- R' eine hydrophobe Gruppe mit mindestens 6 Kohlenstoffatomen ist, enthalten.

14. Feste Partikel nach Anspruch 13, wobei der Wirkstoff aus Schmerzmitteln, Entwöhnungswirkstoffen, Antibiotika, Antikrebsmitteln, zur Behandlung von Diabetes verwendeten Wirkstoffen, Antidepressiva, zur Behandlung von Magenerkrankungen verwendeten Wirkstoffen, zur Cholesterinkontrolle verwendeten Wirkstoffen, zur Behandlung von kardiovaskulären Erkrankungen verwendeten Wirkstoffen, zur Behandlung von psychischen Erkrankungen verwendeten Wirkstoffen oder einem Gemisch dieser Wirkstoffe ausgewählt wird.

15. Wässrige Formulierungen, wässrige Dispersionen von festen Partikeln oder feste Partikel, die einen pharmazeutischen Wirkstoff und eine Emulsion des HASE-Typs enthalten, wobei die Emulsion mindestens ein Copolymer aus den Folgenden enthält:
- (Meth)Acrylsäure,
- einem nicht-wasserlöslichen (Meth)Acrylsäureester-Monomer und
- einem Monomer, das mindestens eine hydrophobe Gruppe mit der folgenden allgemeinen Formel enthält: 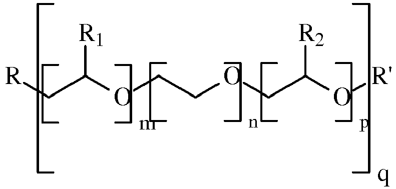 worin:
- m, n, p und q ganze Zahlen unter 150 sind,
- R eine polymerisierbare Vinylfunktion trägt,
- R₁ und R₂ gleich oder verschieden sind und für H oder Alkylgruppen stehen,
- R' eine hydrophobe Gruppe mit mindestens 6 Kohlenstoffatomen ist, zur Verwendung als Mittel mit der doppelten Funktion,
den pharmazeutischen Wirkstoff in saurem Medium zu schützen und diesen in basischem oder alkalischem Medium freizusetzen.

16. Verwendung einer Emulsion des HASE-Typs, wobei die Emulsion mindestens ein Copolymer aus den Folgenden enthält:
- (Meth)Acrylsäure,
- einem nicht-wasserlöslichen (Meth)Acrylsäureester-Monomer und
- einem Monomer, das mindestens eine hydrophobe Gruppe mit der folgenden allgemeinen Formel enthält: 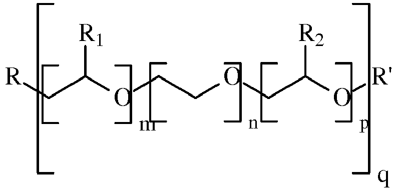 worin:
- m, n, p und q ganze Zahlen unter 150 sind,
- R eine polymerisierbare Vinylfunktion trägt,
- R₁ und R₂ gleich oder verschieden sind und für H oder Alkylgruppen stehen,
- R' eine hydrophobe Gruppe mit mindestens 6 Kohlenstoffatomen ist,
zur Herstellung einer wässrigen Formulierung, einer wässrigen Dispersion von festen Partikeln oder fester Partikel, die einen pharmazeutischen Wirkstoff enthalten.

## Claims

1. Method for manufacturing a formulation containing a HASE emulsion (Hydrophobically Alkali Swellable Emulsion) and a pharmaceutical active ingredient, comprising the steps of:
a) mixing of a HASE emulsion, a pharmaceutical active ingredient and water, said mixture having a pH greater than 5, said HASE emulsion containing at least a copolymer of:
- meth(acrylic) acid,
- a non water-soluble meth(acrylic) ester monomer, and
- a monomer containing at least a hydrophobic group having the general formula: 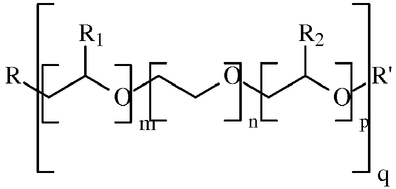 in which:
- m, n, p and q are integers less than 150,
- R has a polymerizable vinylic function,
- R₁ and R₂ are identical or different and represent H or alkyl groups,
- R' is a hydrophobic group comprising at least 6 carbon atoms,
b) precipitation by adjusting the pH to a value less than 3, to obtain a dispersion of solid particles in water,
c) optionally purification, to obtain solid particles.

2. Method according to claim 1, according to which the mixture's pH, during step a), is adjusted by an organic or mineral base.

3. Method according to one of claims 1 or 2, according to which, during step a), 0.1% to 20%, by dry weight of said HASE emulsion, in relation to the total weight of the aqueous formulation obtained after step a), is implemented.

4. Method according to one of claims 1 to 3, according to which, during step a), 0.1% to 20% by dry weight of a hydrophilic or hydrophobic pharmaceutical active ingredient, in relation to the total weight of the aqueous formulation obtained after step a), is implemented.

5. Method according to one of claims 1 to 4, **characterized in that** a strong or somewhat strong acid is implemented during step b).

6. Method according to one of claims 1 to 5, according to which the active ingredient is chosen from among pain relievers, withdrawal active ingredients, antibiotics, anti-cancer medications, active ingredients used in the treatment of diabetes, antidepressants, active ingredients used in the treatment of gastric illnesses, active ingredients used for controlling cholesterol, active ingredients used in the treatment of cardiovascular illnesses, active ingredients used in the treatment of mental illnesses, or a mixture of these active ingredients.

7. Aqueous formulation containing water, a pharmaceutical active ingredient and a HASE emulsion, said HASE emulsion containing at least a copolymer of:
- meth(acrylic) acid,
- a non water-soluble meth(acrylic) ester monomer, and
- a monomer containing at least a hydrophobic group having the general formula: 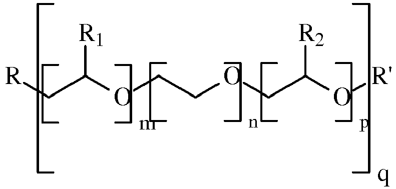 in which:
- m, n, p and q are integers less than 150,
- R has a polymerizable vinylic function,
- R₁ and R₂ are identical or different and represent H or alkyl groups,
- R' is a hydrophobic group comprising at least 6 carbon atoms,
said formulation having a pH greater than 5.

8. Aqueous formulation according to claim 7, containing 0.1% to 20% by dry weight of said HASE emulsion, in relation to its total weight.

9. Aqueous formulation according to one of claim 7 or 8, containing 0.1% to 20% by dry weight of a hydrophilic or hydrophobic pharmaceutical active ingredient, in relation to its total weight.

10. Aqueous formulation according to one of claims 7 to 9, in which the active ingredient is chosen from among pain relievers, withdrawal active ingredients, antibiotics, anti-cancer medications, active ingredients used in the treatment of diabetes, antidepressants, active ingredients used in the treatment of gastric illnesses, active ingredients used for controlling cholesterol, active ingredients used in the treatment of cardiovascular illnesses, active ingredients used in the treatment of mental illnesses, or a mixture of these active ingredients.

11. Dispersion of solid particles in water, said solid particles containing a pharmaceutical active ingredient and a copolymer of:
- meth(acrylic) acid,
- a non water-soluble meth(acrylic) ester monomer, and
- a monomer containing at least a hydrophobic group having the general formula: 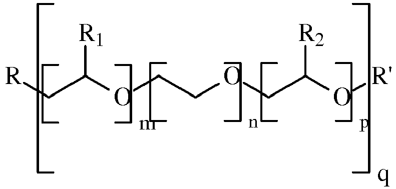 in which:
- m, n, p and q are integers less than 150,
- R has a polymerizable vinylic function,
- R₁ and R₂ are identical or different and represent H or alkyl groups,
- R' is a hydrophobic group comprising at least 6 carbon atoms.

12. Dispersion of solid particles according to claim 11, in which said active ingredient is chosen from among pain relievers, withdrawal active ingredients, antibiotics, anti-cancer medications, active ingredients used in the treatment of diabetes, antidepressants, active ingredients used in the treatment of gastric illnesses, active ingredients used for controlling cholesterol, active ingredients used in the treatment of cardiovascular illnesses, active ingredients used in the treatment of mental illnesses, or a mixture of these active ingredients.

13. Solid particles containing a pharmaceutical active ingredient and a copolymer of:
- meth(acrylic) acid,
- a non water-soluble meth(acrylic) ester monomer, and
- a monomer containing at least a hydrophobic group having the general formula: 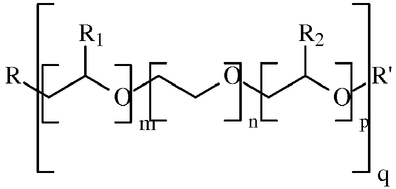 in which:
- m, n, p and q are integers less than 150,
- R has a polymerizable vinylic function,
- R₁ and R₂ are identical or different and represent H or alkyl groups,
- R' is a hydrophobic group comprising at least 6 carbon atoms.

14. Solid particles according to claim 13, in which said active ingredient is chosen from pain relievers, withdrawal active ingredients, antibiotics, anti-cancer medications, active ingredients used in the treatment of diabetes, antidepressants, active ingredients used in the treatment of gastric illnesses, active ingredients used for controlling cholesterol, active ingredients used in the treatment of cardiovascular illnesses, active ingredients used in the treatment of mental illnesses, or a mixture of these active ingredients.

15. Aqueous formulations, aqueous dispersions of solid particles or solid particles, containing a pharmaceutical active ingredient and a HASE emulsion, said emulsion containing at least a copolymer of:
- meth(acrylic) acid,
- a non water-soluble meth(acrylic) ester monomer, and 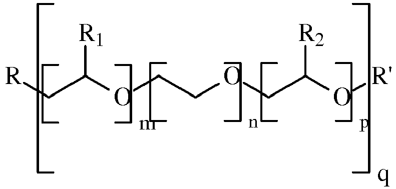
- a monomer containing at least a hydrophobic group having the general formula: in which:
- m, n, p and q are less than 150,
- R has a polymerizable vinylic function,
- R₁ and R₂ are identical or different and represent H or alkyl groups,
- R' is a hydrophobic group comprising at least 6 carbon atoms,
for their use as agent having the dual function of protecting said pharmaceutical active ingredient in an acidic medium and releasing it into a basic or alkaline medium.

16. Use of a HASE emulsion, said emulsion containing at least a copolymer of:
- meth(acrylic) acid,
- a non water-soluble meth(acrylic) ester monomer, and
- a monomer containing at least a hydrophobic group having the general formula: 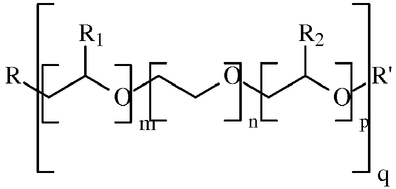 in which:
- m, n, p and q are integers less than 150,
- R has a polymerizable vinylic function,
- R₁ and R₂ are identical or different and represent H or alkyl groups,
- R' is a hydrophobic group comprising at least 6 carbon atoms,
to prepare an aqueous formulation, an aqueous dispersion of solid particles or solid particles, containing a pharmaceutical active ingredient.
